(19) Europäisches Patentamt
European Patent Office
Office européen des brevets

(11) Veröffentlichungsnummer: **0 195 888**
**A2**

(12) # EUROPÄISCHE PATENTANMELDUNG

(21) Anmeldenummer: **86100655.9**

(22) Anmeldetag: **20.01.86**

(51) Int. Cl.⁴: **A 61 K 31/55**

(30) Priorität: **25.01.85 DE 3502365**

(43) Veröffentlichungstag der Anmeldung: **01.10.86 Patentblatt 86/40**

(84) Benannte Vertragsstaaten: **AT BE CH DE FR GB IT LI LU NL SE**

(71) Anmelder: **BOEHRINGER INGELHEIM KG, D-6507 Ingelheim am Rhein (DE)**

(84) Benannte Vertragsstaaten: **BE CH DE FR IT LI LU NL SE AT**

(71) Anmelder: **Boehringer Ingelheim International G.m.b.H, D-6507 Ingelheim am Rhein (DE)**

(84) Benannte Vertragsstaaten: **GB**

(72) Erfinder: **Brantl, Victor, Dr., Matthias-Claudius-Strasse 9, D-6200 Wiesbaden (DE)**
Erfinder: **Bomann, Werner, Dr., Albrecht-Dürer-Strasse 21, D-6507 Ingelheim/Rhein (DE)**
Erfinder: **Schill, Herbert, Dr., Raiffeisenstrasse 12, D-6531 Appenheim (DE)**

(54) **Verwendung von 2-Amino-6-allyl-5,6,7,8-tetrahydro-4H-thiazolo[5,4-d]azepin zur Senkung des Prolactin-Serum-Spiegels.**

(57) Beschrieben ist die Verwendung der 2-Amino-5-allyl-5,6,7,8-tetrahydro-4H-thiazolo [5,4-d] azepin und deren Säureadditionssalze bei der Verminderung des Prolactinspiegels im Blut.

EP 0 195 888 A2

EZEICHNUNG GEÄNDERT
siehe Titelseite





In den Belgischen Patentschriften 684 415 und 771 330 werden u.a. Thiazolo- und Oxazolo-Derivate der allgemeinen Formel

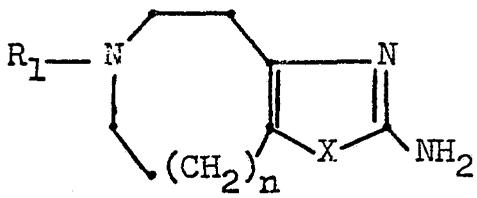

in der

$R_1$ ein Wasserstoffatom, eine gegebenenfalls durch eine Hydroxylgruppe substituierte Alkylgruppe mit 1 bis 4 Kohlenstoffatomen, eine gegebenenfalls durch ein Halogenatom, eine Methyl- oder Methoxygruppe substituierte Benzylgruppe oder eine Allylgruppe,

n die Zahl 2 oder auch 1, wenn X ein Schwefelatom darstellt, und

X ein Sauerstoff- oder Schwefelatom bedeuten, und deren physiologisch verträgliche Säureadditionssalze mit anorganischen oder organischen Säuren beschrieben.

Aus den Belgischen Patentschriften 684 415 und 771 330 ist bekannt, daß die Verbindungen der allgemeinen Formel I und deren physiologisch verträgliche Säureadditionssalze wertvolle pharmakologische Eigenschaften aufweisen. So weisen die in der Belgischen Patentschrift 684 415 beschriebenen Verbindungen insbesondere eine analgetische, sedative, antitussive, antipyretische und antiphlogistische Wirkung und die in der Belgischen Patentschrift 771 330 beschriebenen Verbindungen je nach ihrer Substitution insbesondere eine blutdrucksenkende, sedative, hustenstillende und/oder antiphlogistische Wirkung auf. Desweiteren geht aus den obengenannten Patentschriften hervor, daß die Thiazolo-Derivate der allgemeinen Formel I, in der $R_1$ eine Alkylgruppe mit 1 bis 4 Kohlenstoffatomen oder eine Allylgruppe, n die

Zahl 2 und X ein Schwefelatom bedeuten, insbesondere eine blutdrucksenkende Wirkung und die Oxazolo-Derivate der allgemeinen Formel I, in der $R_1$ ein Wasserstoffatom, eine gegebenenfalls durch eine Hydroxygruppe substituierte Alkylgruppe mit 1 bis 4 Kohlenstoffatomen oder eine Allylgruppe, n die Zahl 2 und X ein Sauerstoffatom bedeuten, insbesondere hustenstillende Eigenschaften aufweisen.

Es ist aus der EP-A1-0 005 732 bekannt, daß die Verbindungen der obigen allgemeinen Formel I, in der n die Zahl 2 darstellt, auch eine antanginöse Wirksamkeit aufweisen.

Zur medizinischen Anwendung wird sowohl in der BE-PS 771 330 und der BE-PS 684 415 als auch in der EP-A1-0 005 732 ausschließlich deren endogene Applikation beschrieben.

Ferner ist aus der US-Patent Nr. 4 400 378 bekannt, daß die Verbindungen der allgemeinen Formel I auch eine Antiglaucoma Wirksamkeit aufweisen.

Es ist bekannt, daß 6-Allyl-2-amino-5,6,7,8-tetrahydro-4H-thiazolo-[4,5-d]azepin-dihydrochlorid (nachfolgend als B-HT 920 bezeichnet) selektiv agonistisch auf Dopamin-Autorezeptoren (DA-Autorezeptoren) wirkt (Anden et al., Naunyn-Schmiedeberg's Arch. Pharmacol., 321, 100-104. 1982). Weiterführende Untersuchungen mit B-HT 920 zeigten, daß diese Substanz in vivo die endogene Dopaminsynthese im Gehirn hemmt. (Anden et al., Acta pharmacol. et toxicol., 52, 51-56, 1983). Beim Dopamin handelt es sich bekanntlicherweise um eine Schlüsselsubstanz bei der Regulation des Hormons Prolaktin (PRL): Dopamin ist der sogenannte "Prolactin Inhibiting Factor" (PIF), d.h. Dopamin (= PIF) oder andere Dopaminagonisten hemmen die Freisetzung von PRL aus der Hypophyse. Eine Verminderung des Dopamins müßte somit zu einem Überschießen der PRL-Sekretion führen, da der hemmende Faktor wegfällt (Selecta, Nr. 46, 15.11.1982, S. 4344).

Es ist bekannt, daß die literaturbekannte DA-Autorezeptor-Agonisten

TL-99 und 3-PPP eine Prolactin-Hemmung-Wirkung haben (Gudelsky, European Journal of Pharmacology 90, (1983) 423, 425).

Jedoch TL-99, 3-PPP und auch der DA-Autorezeptor Agonisten Apomorphin sind für Vergleiche mit B-HT 920 nicht geeignet, da die genannten Stoffe nicht selektiv als DA-Autorezeptoragonisten wirksam sind. Gudelsky et al. berichten im Europ. J. Pharmacol, 90, 423-425, 1983, daß TL-99 und 3-PPP vorzugsweise als DA-agonisten wirken, also nicht als selektive DA-Autorezeptoragonisten. Pastor et al berichten im Europ. J. Pharmacol. 87 (1983) 459-464, daß TL-99 möglicherweise eine postsynaptische Wirkung hat, d.h. eine Wirkung an den DA-Rezeptoren. Weiterhin wirken die Stereoisomeren der Substanz 3-PPP unterschiedlich auf die verschiedenen DA-Rezeptoren. (Koch et al., Europ. J. Pharmacol., 92, 279-283, (1983). Apomorphin wirkt bekanntermaßen ebenfalls sowohl auf DA-Rezptoren wie auch auf DA-Autorezeptoren.

Goodale et al. schreibt im Science Vol. 210, 05. Dezember 1980, daß ein Mittel welche eine postsynaptische Wirkung hat, zur Behandlung Hyperprolactine indiziert ist.

Überraschenderweise wurde nun gefunden, daß 6-Allyl-2-amino-5,6,7,8-tetrahydro-4H-thiazolo-[4,5-d]azepin (nachfolgend als Verbindung A bezeichnet) und deren physiologisch verträgliche Säureadditionssalze mit anorganischen oder organischen Säuren eine therapeutisch notwendig senkende Wirkung auf den Prolactin-Serum-Spiegel (PRL) aufweisen.

Zur Verminderung des PRL-Spiegels im Blut lassen sich die Verbindungen A und deren geeignete Säureadditionssalze in die üblichen galenischen Zubereitungsformen für oral, parenteral, rektal oder transdermale Anwendung einarbeiten. Die orale Einzeldosis am Menschen normalerweise ist zwischen 2,5 µg und 350 µg, vorzugsweise zwischen 100 µg und 250 µg; bei Mehrfachapplikation (z.B. 3x täglich) können die Tagesdosen normalerweise zwischen 15-900 µg betragen - vorzugsweise zwischen 30-750 µg. Die parenteralen und rektalen Dosen können im gleichen Bereich wie die oral anzuwendenden Substanzmengen liegen.

Verbindung A und deren geeignete Säureadditionssalze, inbesondere B-HT 920 können aufgrund ihrer PRL senkenden Eigenschaften immer dann eingesetzt werden, wenn eine Verminderung des PRL-Spiegels im Blut indiziert ist. Dies kann insbesonders bei folgenden Krankheitsbildern therapeutisch zweckmäßig sein:

1.) Hypophysentumore, Suprselläre Tumore (Craniopharyngeom), Unterbrechung der hypophysäuren Portalgefäße,

2.) Vermehrung PRL-produzierender Zellen (Schwangerschaft, Hyperplasie, Prolaktinom),

3.) Bei Überwiegen PRL-sekretionsfördernder Faktoren über PIF (Hypothyreose),

4.) Zyklusstörungen:
- Amenorrhoe mit oder ohne Galaktorrhoe
- Oligomenorrhoe
- PRL-bedingte Pertilitätsstörungen
- Corpus-luteum Insuffizienz
- Praemenstruelles Syndrom
- Dysmenorrhoe
- Anovulatorische Zyklen
- Post-pill Amenorrhoe

5.) Brustdrüsen-Krebs (Mamma-Ca)

6.) Zervix-Dysplasien

7.) Bei medikamentös bedingter Blockade der DA-Rezeptoren bzw. Hemmung der DA-Sekretion: Kombination von B-HT 920 mit Arzneimitteln, die mit einer Erhöhung des PRL einhergehen (z.B. Neuroleptika von Phenothiazin- oder Butyrophenontyp; Antiemetika, z.B. Metoclopramid).

8.) Laktationshemmung (primäres und sekundäres Abstillen)

9.) Puerperale Mastitis

10.) PRL-bedingter Hypogonadismus.

Beispielsweise wurde die Verbindung B-HT 920 auf ihre Wirkung auf die PRL-Senkung wie folgt untersucht:

Die Versuche wurden mit 6 gesunden männlichen Probanden im Alter von 24 bis 34 Jahren durchgeführt, jeder Proband wurde mit 150 µg B-HT 920 Dosis gegeben.

Blutproben wurden in 0, 1, 2, 3 und 5 Stunden Zwischenräumen an jedem Probanden genommen. Durch die bekannte PROL-RIA-100 Radioimmunologische Methode des Instituts National des Radioelements, 6220 Fleurs, Belgien wurde die in der Probe Prolakten Spiegel bestimmt. Die nachfolgende Tabelle enthält die gefundenen Werte:

| | Prolactin-Serum-Spiegel mg/L | | | | | |
|---|---|---|---|---|---|---|
| Proband / Std. nach Verabreichung | 1 | 2 | 3 | 4 | 5 | 6 |
| 0 | 3.33 | 3.86 | 4.22 | 5.04 | 5.00 | 6.59 |
| 1 | 2.56 | 2.85 | 4.15 | 4.52 | 7.19 | 7.59 |
| 2 | 0.52 | 0.69 | 4.92 | 7.89 | 1.37 | 1.57 |
| 3 | 2.52 | 0.52 | 1.26 | 7.19 | 3.43 | 1.39 |
| 5 | 3.26 | 2.25 | 1.85 | 4.11 | 2.89 | 4.33 |

Desweiteren weist die Verbindung A und ihre physiologisch verträglichen Säureadditionssalze eine gute Verträglichkeit auf, beispielsweise weist B-HT 920 an der Maus eine orale $LD_{50}$ von 455 mg/kg auf.

Die nachfolgenden Beispiele sollen die Erfindung näher erläutern:

Die nachfolgenden Beispiele beschreiben die Herstellung einiger pharmazeutischer Zubereitungsformen.

## Beispiel I

Dragéekern

Zusammensetzung:

1 Dragéekern enthält:

| | |
|---|---|
| B-HT 920 | 50 µg |
| Milchzucker | 38,45 mg |
| Maisstärke | 10,0 mg |
| Gelatine | 1,0 mg |
| Magnesiumstearat | 0,5 mg |
| | 50,0 mg |

Herstellungsverfahren:

Die Mischung der Wirksubstanz mit Milchzucker und Maisstärke wird mit einer 10 %igen wäßrigen Gelatinelösung durch Sieb 1 mm granuliert, bei 40°C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

Kerngewicht: 50 mg
Stempel: 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 100 mg

Beispiel II

Suppositorien

1 Zäpfchen enthält:

| | |
|---|---|
| B-HT 920 | 100,0 µg |
| Zäpfchenmasse (z.B. Witepsol W 45) | 1690,0 mg |

Herstellungsverfahren:

Die feingepulverte Substanz wird mit Hilfe eines Eintauchhomogenisators in die geschmolzene und auf 40°C abgekühlte Zäpfchenmasse eingerührt. Die Masse wird bei 35°C in leicht vorgekühlte Formen ausgegossen.

Beispiel III

Ampullen mit 200 µg B-HT 920

1 Ampulle enthält:

| | | |
|---|---|---|
| B-HT 920 | | 200 µg |
| Zitronensäure | | 7,0 mg |
| Natriumphosphat sek. · 2 $H_2O$ | | 3,0 mg |
| Natriumpyrosulfit | | 1,0 mg |
| Dest. Wasser | ad | 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

Abfüllung: in braune Ampullen unter Schutzbegasung
Sterilisation: 20 Minuten bei 120°C

Die Herstellung und Abfüllung der Ampullenlösung muß in abgedunkelten Räumen vorgenommen werden.

## Beispiel IV

Dragées mit 1 mg B-HT 920

1 Dragéekern enthält:

| | |
|---|---|
| B-HT 920 | 100 µg |
| Milchzucker | 36,0 mg |
| Maisstärke | 12,4 mg |
| Gelatine | 1,0 mg |
| Magnesiumstearat | 0,5 mg |
| | 50,0 mg |

Herstellungsverfahren:

Analog Beispiel I.
Kerngewicht: 50 mg
Stempel: 5 mm, gewölbt
Dragéegewicht: 100 mg

Beispiel V

Dragées mit 0,2 mg B-HT 920

1 Dragéekern enthält:

| | |
|---|---|
| B-HT 920 | 0,2 mg |
| Digoxin | 0,25 mg |
| Milchzucker | 66,55 mg |
| Kartoffelstärke | 25,0 mg |
| Polyvinylpyrrolidon | 2,0 mg |
| Magnesiumstearat | 1,0 mg |
| | 120,0 mg |

Herstellungsverfahren:

Die intensive Mischung der Wirksubstanz mit Milchzucker und Kartoffelstärke wird mit einer 10 %igen Lösung des Polyvinylpyrrolidons in Äthanol durch Sieb 1,5 mm granuliert, bei 40°C getrocknet und nochmals durch Sieb 1,0 mm gerieben. Das so erhaltene Granulat wird mit Magnesiumstearat gemischt und zu Dragéekernen verpreßt.

Kerngewicht: 95 mg
Stempel: 7 mm, gewölbt

Die so hergestellten Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 175 mg.

Beispiel VI

Gelatine-Steckkapseln mit 300 µg B-HT 920

1 Kapsel enthält:

| | |
|---|---|
| B-HT 920 | 300 µg |
| Codeinphosphat | 10,0 mg |
| Weinsäure | 3,0 mg |
| Maisstärke | 86,7 mg |
| | 100,0 mg |

Herstellungsverfahren:

Die Substanzen werden intensiv gemischt und in Opak-Kapseln geeigneter Größe abgefüllt.
Kapselfüllung: 100 mg

## Patentansprüche:

1. 2-Amino-6-allyl-5,6,7,8-tetrahydro-4H-thiazolo[5,4-d]azepin und dessen Säureadditionssalze zur Verwendung bei der Verminderung des Prolactinspiegels im Blut.

2. Verbindungen gemäß Anspruch 1 zur Herstellung von zur Verminderung des Prolactinspiegels im Blut geeigneten Arzneimitteln.

3. Arzneimittel gemäß Anspruch 1, dadurch gekennzeichnet, daß die Einzeldosis 2,5 bis 350 µg, vorzugsweise 100 bis 250 µg, einer Verbindung gemäß Anspruch 1 beträgt.